# EUROPEAN PATENT APPLICATION

(11) **EP 0 884 594 A2**
(43) Date of publication of application: **16.12.1998**
(21) Application number: 98110162.9
(22) Date of filing: 04.06.1998
(51) Int. Cl.: G01N 33/558

(54) **Analyte-fixation immunochromatographic device**

(30) Priority: 13.06.1997 IT MI971406
(71) Applicant: Torelli, Giorgio, 20127 Milan (IT)
(72) Inventor: Torelli, Giorgio, 20127 Milan (IT)
(74) Representative: Gervasi, Gemma, Dr.

(57) **Abstract**

Described herein is a new immunochromatographic device for the detection of analytes in a biological sample, comprising a chromatographic membrane provided with a support, at one end of which is applied, possibly by an absorbent pad in contact with the said membrane, a marked agent (antibody or conjugated antigen) that is specific for the analyte. After seeding and hence fixing the biological sample in an intermediate area (i.e., between the two ends) of the chromatographic membrane, the user sets an appropriate buffer ahead of the marked agent, which is then drawn along chromatographically up to the band where the sample is laid, with consequent conjugate-analyte immunological reaction, which may be visually detected in the form of a coloured band.

## Description

### Scope of invention

The present invention regards a new device for immuno-chromatographic analysis which is particularly advantageous in that it enables semi-quantitative determinations of one or more analytes that may be present in a biological sample.

### State of the art

Various immunochromatographic diagnostic tests are known in the state of the art for the determination of the presence of analytes, based on the principle described below.

The biological fluid under examination, generally blood, plasma, serum, urine or cerebrospinal fluid (CSF), is deposited at the end of a chromatographic strip (generally made of cellulose acetate or cellulose nitrate) and migrates throughout the strip by chromatographic diffusion.

The analyte that is to be detected (antigen) moves with the biological liquid and is captured immunologically by a specific antibody, which has previously been immobilized on the chromatographic membrane in the form of a band.

A second antibody that is specific for the analyte, marked with a colloidal metal or other coloured substances, is deposited, but not immobilized, at a short distance after the site of deposition of the sample. This second antibody is drawn along in the chromatographic migration of the sample itself, so binding to the analyte that is to be detected, if the latter is present in the sample. The complex that is thus formed during migration across the chromatographic strip is in this way blocked by the immobilized capturing antibody and gives rise to the formation of a coloured band (detection band). The American patent USP 4,855,240 (Derwent extract) describes a solid-phase device for the qualitative determination of the presence of various analytes, which exploits the principle outlined above.

Generally the marking agent consists of a coloured substance, the presence of which can be detected visually in a convenient manner and without the aid of additional apparatus.

For example, the American patent USP 4,703,017 (Derwent extract) describes a (non-chromatographic) solid-phase immunological assay in which the marked antibody, which is specific for the analyte that is to be revealed, is conjugated to particles of coloured substances that are easily observable to the naked eye.

These coloured substances may be particles of colloidal metal coated with the conjugated agent (see, for instance, USP 4,313,734 - Derwent extract), or else may be non-metallic colloidal substances (for example, selenium, tellurium or sulphur), bound to or coated with the conjugated antibody (such as those described in USP 4,954,452 - Derwent extract), or finally are particles of coloured latex, to which the marked antibody is made to adhere.

These diagnostic methods present, however, the disadvantage of enabling only cut-off qualitative analyses; i.e., they make it possible only to obtain either a positive result or a negative result as regards the presence or absence of the analyte being sought. Instead they are not able to carry out a quantitative or semi-quantitative determination of the analytes.

The American patent USP 4,435,504 (Derwent extract) describes a semi-quantitative determination of an analyte, using immuno-enzymatic techniques that employ a number of capturing agents specific for the said analyte, and a marked conjugate (by means of enzymatic marking). More specifically, the capturing agents are immobilized on a chromatographic strip in bands which are progressively further away from the end of the chromatographic strip on which the marked conjugate is applied. After the said end has been set in contact with the sample of liquid under examination, in association with an appropriate solvent, the analyte contained therein binds to the marked agent, and the complex of the two that has formed migrates across the chromatographic strip until it reaches the various bands of capturing agents, with which it interacts. The level of the marking front thus obtained, which can be detected by means of electromagnetic-radiation analysis, may be directly correlated to the quantity of analyte present in the sample being examined. Obviously, marking with enzymes does not enable a detection by the naked eye, but involves complex procedures and equipment.

All the immunodiagnostic methods described above present the disadvantage of requiring, in addition to the presence of a marked agent, the presence of one or more capturing agents that are specific for the analyte sought, generally monoclonal antibodies. These capturing agents must be immobilized on the chromatographic strip in a uniform manner and limited to the precise area in which the detection is to take place. The said immobilization involves the use of costly techniques and involves considerable operating difficulties.

### Summary

The applicant has found an immuno-chromatographic device for *in vitro* determination of one or more analytes, comprising a chromatographic membrane provided with a support, at one end of which is applied (not immobilized), possibly by an absorbent pad that is in contact with the said chromatographic membrane, a marked agent which is specific for the analyte. This device is characterized in that it does not present any capturing agent, which is specific for the analyte, immobilized on the chromatographic membrane. It does not in fact require the use of agents that are able to block the analyte in the detection area, as instead is required by devices known to the state of the art.

The said device may possibly present one or more absorbent pads in contact with the other end of the chromatographic membrane. It may moreover present a control band set ahead of this latter end of the chromatographic membrane.

The present device enables direct use by the user in a rapid and simple way, and affords the possibility of carrying out semi-quantitative analyses having high sensitivity and accuracy, even starting from very small amounts of the biological sample that is to be analyzed.

The present invention moreover regards an immuno-chromatographic method for the determination of one or more analytes which uses the said device. This method comprises:
i) application of a biological sample that might contain the analyte to be sought on the device of the invention, in an intermediate area (i.e., between the two ends) of the chromatographic membrane;
ii) elution of the marked agent, which is applied to one end of the chromatographic membrane, possibly by means of an absorbent pad, using a suitable eluent, which is able to cause the said marked agent to migrate up to an intermediate area in which the analyte has been applied;
iii) detection of the complex resulting from the interaction between the said analyte and the said marked agent, in the intermediate area in which the analyte has been applied.

Finally, the present invention regards an immunochromatographic kit comprising the above-mentioned device, a suitable eluent and, possibly, a positive control solution and a depositor for application of the sample.

### Detailed description of the invention

The characteristics and advantages of the immunochromatographic device, of the method for the use thereof, and the corresponding kit, according to the present invention, will be further illustrated in the course of the following detailed description.

The analytes that may be detected using the device of the invention may be lipopolysaccharides, plasma proteins or proteins coming from other biological samples, such as hormones, enzymes, immuno-globulins, allergens, viruses, viral subunits, bacterial extracts, toxins, extracts of mitochondria, and extracts of cell nuclei and cellular membranes, or other proteids, such as DNA, RNA, chromosomes and genes.

The device of the invention may be used for the detection of hormones, such as HCG (pregnancy test), FSH (pre-ovulation test), LH (ovulation test), TSH, T3, T4, prolactin (HPRL), etc.

The test may moreover determine the presence of infections, in the diagnosis of HIV and HIV2, HCV, HBV (by detecting HBcAb, HBclgM, HBeAb, HBeAg, HBsAb, or HBsAg), tuberculosis and mononucleosis, as well as the presence of Salmonella, Escherichia coli, H. Pylori (by detecting IgG or IgA), Rubella (by detecting IgG or IgM), Chlamydia, Streptococcus (Groups A and B), etc.

In addition, various protein analytes may be detected, for example, alpha foetoprotein (AFP), prostate specific antigen (PSA), and cardiac and tumoral markers, in the diagnosis of various carcinogenic forms, in addition to the presence of occult haemoglobin in the faeces.

The device of the invention proves particularly advantageous in the determination of the presence in urine of the Bence Jones protein, the kappa and lambda light chains, or mixtures of these, in the diagnosis of Bence Jones proteinuria. In fact, up to the present day, no immunochromatographic tests are known to the state of the art which are able to determine the presence of the said proteins.

In addition, the device of the invention enables detection, without the need for particular apparatus, of the presence of drugs, metabolites, pesticides and polluting substances; it is possible, for example to assess the use of narcotics or the abuse of drugs, such as amphetamines, methamphetamine, barbiturates, benzodiazepines, cannabis-type drugs, marijuana (THC), cocaine, methadone, morphine, heroin, opiates, phencyclidine and their metabolites.

The above-mentioned analytes are preferably contained in body fluids, such as urine, CSF, blood, plasma and serum, or other biological samples, such as mucus and faeces. The samples may be applied as such to the device according to the present invention or may undergo appropriate preventive treatments, such as extraction, where the biological sample consists of faeces.

The said device also enables analysis of coloured or turbid substances, whether in the form of a solution or in the form of a suspension, without this causing any interference with the detecting system.

The device of the invention does not require large amounts of the samples to be analyzed, in so far as quantities in the order of the microliter are at times sufficient.

The quantity of the sample is suitably varied according to the size and shape of the device used.

By the term "marked agent" is meant a coloured substance (or a substance that is in any case detectable by the naked eye) which is able to recognize the analyte that is to be detected and bind to it in a specific way. More in particular, the said agent may consist of an immunological component conjugated to coloured substances, bound either directly or indirectly to the said substances.

The said immunological component is chosen preferably from the group made up of haptens, antigens and antibodies (preferably monoclonal ones).

The said coloured substances may be colloidal particles of metals, such as gold, silver and platinum, or derivatives of metals. Alternatively, the said coloured substances may be carbon, coloured latexes, or non-metallic colloidal particles, such as particles of sulphur, selenium or tellurium.

The marked agent is preferably applied to the chromatographic medium by means of an absorbent pad, such as absorbent paper or glass fabric, overlying, and in contact with, the chromatographic membrane itself. The absorbent pad not only has the purpose of providing a support for the marked conjugate, but also functions as a vehicle for the eluent which should cause its advance across the chromatographic strip.

By "chromatographic membrane" is meant a porous, solid support that is able to guarantee the passage of the eluent and, preferably, consists of a membrane made of cellulose acetate, nitrocellulose, nylon, or other materials suitable for thin-layer chromatography. These materials are provided with supports according to techniques known to the state of the art.

The other end of the said chromatographic membrane provided with support is possibly overlaid with an absorbent pad that is in contact with the membrane. This pad preferably consists of absorbent paper and is useful for promoting the chromatographic migration of the eluent and of the various reagents across the chromatographic membrane itself.

The analytical device of the invention may take the form of a dipstick, without any stiff envelope, which is to be dipped directly into the buffer contained in an appropriate container.

The said device may moreover take the form of a strip housed in a suitable rigid container, for example one made of plastic, having a well for immission of eluent, to be used horizontally by causing the marked agent to flow across the chromatographic membrane provided with support (card). The lid of the said card preferably presents an intermediate opening for the application, on the chromatographic strip, of the biological sample and for control of reagent flow.

The above devices, whether in the form of a card or in the form of a dipstick, may be advantageously kept in suitable containers and may carry the data regarding the user or similar useful comments or notes.

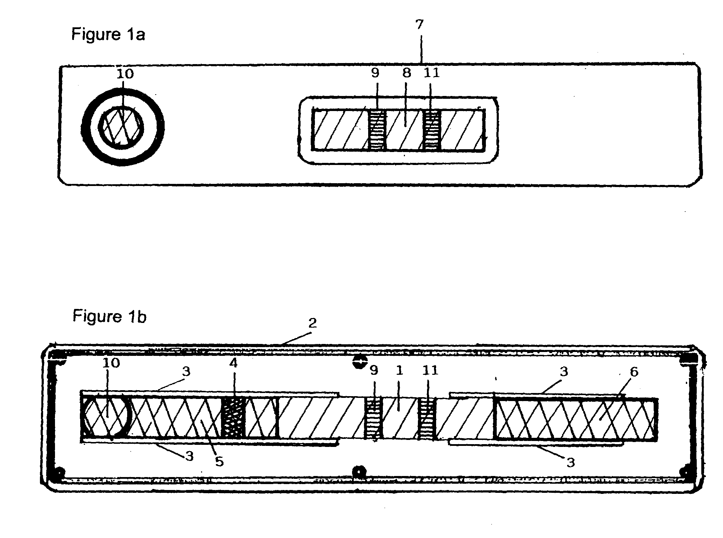

**Figure 1a** presents a plan view of the external part of the device according to the invention, in the form of a card, according to a preferred embodiment, whilst
**Figure 1b** is a plan view of the internal part of the card. The device consists of a chromatographic membrane provided with a support 1 in the form of a strip, attached to a rigid support 2 by means of internal side guides 3. The marked agent (band 4) is applied to an absorbent pad 5, in contact with one end of the chromatographic membrane 1, which moreover presents an absorbent membrane 6, in contact with the other end. On the said rigid support 2 is sealed a lid 7 presenting a rectangular window 8, which enables deposition of the sample to be analyzed at the band 9 (detection band) and control of reagent flow, and a round well 10, which enables application of the eluent on the chromatographic strip 1. A control band 11 is moreover present.

In fact, the device according to the invention may present a control band located between the intermediate area of sample deposition and the other end of the chromatographic membrane provided with support. On the said band is immobilized an analyte or an analogue of the latter which is able to yield with certainty a positive response when it comes into contact with the marked agent. At the end of the test, failure of colouring of the control band is an index of the deterioration of the membrane, and hence of the failure of the device of the invention to operate.

The device of the invention may present an applier in the intermediate area of the chromatographic membrane provided with support in which the sample to be examined is to be deposited. The function of the said applier may be that of housing the biological material (blood, mucus, etc.) in which the sample of liquid to be analyzed is contained. A filter may moreover be located on the chromatographic membrane provided with support, in order to prevent the accumulation of particulate matter on the chromatographic medium.

The device according to the invention presents numerous advantages over techniques known to the state of the art.

In the first place, it does not require the use of capturing agents, which are instead indispensable in the devices so far known. In the state of the art, these capturing agents, which are immobilized on the chromatographic medium at the site of the test band, are necessary for blocking the possible analyte-marked agent complex during migration across the chromatographic strip. The elimination of the capturing agent involves a considerable economic saving, in addition to avoiding laborious and complex techniques of immobilization.

In the second place, the invention makes possible the performance of semi-quantitative determinations without the aid of additional instruments or calibrations. In fact, it is possible to deposit the sample to be analyzed in various intermediate areas (bands) of the chromatographic membrane provided with support, at increasing dilutions. The positivity to a greater dilution represents the index of the concentration of the analyte in the sample. In fact, if the sensitivity of the diagnostic test is known, it will be possible to know the concentration of analyte corresponding to the band up to which marking is detectable. This determination is not allowed using chromatographic methods known to the state of the art, except when various tests are employed at the same time.

The test may be carried out directly and in a simple and rapid way by any user, even an inexpert one, and yields results that are clear, easily interpretable, accurate and reproducible. The performance of the test, which presents high sensitivity, requires a time that may range from 5 to 10 minutes. The use of special containers or means of transfer of the liquid samples to be analyzed is not required, and the operation of seeding the sample on the chromatographic medium is particularly simple and fast.

Finally, to carry out the above analysis, very small quantities, i.e., in the order of microliter, of the biological sample to be analyzed are sufficient. Consequently, this method may be advantageously applied also in the case where large amounts of sample are not available.

The immunochromatographic method according to the present invention exploits the use of the analytic device described above, on which the marked agent has previously been deposited (not immobilized), at one end of the chromatographic membrane provided with support.

During performance of the analysis, a given quantity of the sample to be examined is deposited directly by the user in an intermediate area of the chromatographic membrane, where the detection band will be coloured in the case of positive result.

After seeding of the sample, the latter is preferably dried, for example by means of a jet of hot air (using a hot-air blower).

The user then deposits an appropriate amount of eluent at one end of the chromatographic membrane provided with support so that the eluent will migrate across the chromatographic strip from this end to the other end, carrying along with it the marked agent.

The said eluent may be an organic or inorganic buffer (borate, PBS, Tris·HCl, etc.) containing one or more surfactants, such as Triton X® (Rohm and Haas Company), Tween 20® (ICI Americas Inc.), PVP, PVE, and Brij® (ICI PLC). The choice of the buffer will vary according to the analyte to be sought and the marked agent, in such a way that both will advance across the chromatographic strip in a homogeneous manner. In the case where the presence of the Bence Jones protein is to be determined in the urine, a PBS buffer with physiological pH containing Tween 20 at 0.5-5 wt%, Triton X at 0-5 wt% and PVP at 0-5 wt% is preferably used.

The amount of eluent is suitably varied according to the size and shape of the device used.

When the marked agent reaches the intermediate area where the biological sample containing the analyte sought has been deposited, it interacts with the latter, generating a coloured band that may be detected by the naked eye (result of test, positive). In the case, instead, where the biological sample does not contain the analyte sought, no formation of a detection band is observed (negative result).

In the event of a positive response, the intensity of the colouring that has developed on the detection band is proportional to the concentration of the analyte-marked agent complex (depending upon the amount of analyte present in the sample analyzed).

As described above, this method offers the possibility of carrying out not only the traditional cut-off qualitative analysis, but also semi-quantitative determinations. In the latter case, the user must seed the sample to be analyzed a number of times in different intermediate areas (bands) of the chromatographic membrane at progressively increasing, and in any case well determined, dilutions. By observing up to which band the marking is detectable, the concentration of the analyte in the sample under examination will be directly determined, given that the sensitivity of the analytical test is known a *priori*.

According to this method, the biological sample may undergo suitable pretreatments, before being seeded, such as an extraction phase, which is useful in the case where the sample consists of faeces or mucus, these not being directly applicable as such to the device of the invention.

Finally, the present invention regards an immunochromatographic kit comprising the analytical device described above, a suitable eluent, and, possibly, a positive control solution.

The said kit may moreover contain a depositor, such as a microcapillary device, a micropipette or an "electrophoresis-type" depositor (consisting of a double metal strip), for seeding the sample on the device of the invention, given that small quantities of sample must be seeded.

In the case where the biological samples to be analyzed have to undergo pretreatments, such as an extraction phase, the said kit may advantageously contain an appropriate wad for taking the biological sample; this is useful in the case where the sample must be taken from the throat, vagina, cervical canal or urethral canal. In this case, the kit may also comprise a test tube provided with dropper, containing a suitable extraction liquid in which the wad with which the sample has been taken is immersed.

In order to provide a non-limiting illustration of the present invention, the following example is given.

### Determination of Bence Jones protein and kappa and lambda chains in urine

An immunochromatographic analysis was carried out using the device and method according to the present invention, with the purpose of determining the presence, in a sample of urine, of the Bence Jones protein or, separately, of the kappa and lambda chains.

### Materials and Methods

Sample: The test was conducted using diluted urine, without prior centrifugation or filtration being carried out (urine generally presents a stability of 7 days at 2-8°C, whereas it is stable for several months at -20°C).

Solid phase: A chromatographic membrane made of cellulose nitrate was glued to a solid polyvinyl chloride (PVC) support. At one end of the membrane a pad of glass fabric was glued, on which the conjugated antibody was subsequently deposited. The latter was marked with colloidal gold (mixture of goat anti-human kappa conjugated to colloidal gold, Code CG 1589, goat anti-human lambda conjugated to colloidal gold, Code CG 1599, American Qualex, 1997).

Before deposition of the marked conjugated antibody, the card was soaked with PBS at a roughly physiological pH, and then dried. After depositing of the marked conjugate, the latter was dried.

At the other end of the membrane, absorbent paper was laid which had the function of absorbing the chromatographic material.

The solid phase thus obtained was used as such, the first end being immersed in a well containing a suitable quantity of buffer (dipstick). In a second case, the solid phase described above was placed in a pre-moulded box (card), which presented a special hole for the addition of the buffer (the device obtained was similar to the one shown in Figure 1).

Control band: On the filtering membrane, at a site corresponding to the end part of the window in the case of the card, was deposited anti-sheep goat antibody, IgG fraction, SILENUS, Code UC, 1997, capable of binding the marked agent.

Eluent: A buffer comprising PBS, pH 7.4 (±3), Tween 20 at 1.5 wt%, Triton X 100 at 0.5 wt%, and PVP at 2.5 wt% was used as eluent.

Sample depositor: A depositor consisting of a double metal strip to which 1 µl of urine was bound by capillarity was used and was subsequently deposited on the chromatographic membrane.

### Procedure

The reagents and samples were used at room temperature. A sample of the urine under examination having a volume of 1 µl was deposited at the centre of the window of the card, as shown in Figure 1, or at the centre of the dipstick, below the control band. In order to achieve good sensitivity, depositing of the sample was carried out at a relative humidity of not less than 40-45%. The deposited sample was then dried by means of a jet of hot air from a hot-air blower.

In the case of the card, 5 drops of eluent were added in the well, and the card was laid on a horizontal surface for 10 minutes. In the case of the dipstick, 5 drops of eluent were put in a suitable container, and the dipstick was immersed standing in this container at an angle of 70-80°, for 10 minutes.

### Procedure for contrast radiographic analyses

In order to carry out contrast radiographic analyses, generally a concentration of the Bence Jones protein in the urine of not higher than 50-100 mg/dl is required by the radiologist. Consequently, the urine sample was diluted 1:10 (1 part of urine to 9 parts of physiological solution). Using a micropipette or a linear depositor, the urine was then seeded on the chromatographic membrane, just below the control band. Then the procedure was carried out as described above. The sensitivity obtained was approximately 50-100 mg/dl.

### Double cut-off procedure

In order to achieve both high and low levels of sensitivity, 1 µl of urine diluted as described previously was deposited, and 1 µl of undiluted urine was deposited just above the site where the diluted urine was deposited. The procedure followed was then the same as the one described above.

### Results

The formation of a single band corresponding to the end part of the window (control band) was indicative of the fact that the concentration of the kappa and lambda chains in the urine was lower than the cut-off threshold (negative result). The possible presence of the Bence Jones protein in the sample analyzed instead caused a coloured band (test band) to form in the intermediate area of the chromatographic strip in which the urine sample had been seeded. More specifically, if the concentration of kappa and lambda chains in the urine was higher than 5 mg/ml (undiluted urine) or higher than 50 mg/ml (urine diluted 1:10) there was seen to form, within 10 minutes, a coloured band where the sample had been seeded.

Sensitivity: For non-treated urine, the sensitivity was approximately 5-10 mg/dl; for urine diluted 1:10 with physiological solution, the sensitivity was 50-100 mg/dl.

### Expected values

Normal urine yielded a negative signal even when the test was used for high-sensitivity analyses. In the case of positive result due to renal damage (especially of a tubular nature) or due to overstepping of the maximum tubular load (presence of myeloma), the double band was, instead, detected (positive result).

In the case of analyses carried out for contrast radiographic purposes, negative results should emerge at a dilution of 1:10.

## Claims

1. Immunochromatographic device for *in vitro* determination of one or more analytes, comprising a chromatographic membrane provided with a support, at one end of which a marked agent is applied which is specific for the said analyte, characterized in that it does not present any capturing agent, specific for the analyte, immobilized on the chromatographic membrane.

2. Device according to Claim 1, characterized in that the said marked agent is applied on an absorbent pad in contact with one end of the chromatographic membrane provided with support.

3. Device according to Claim 1, characterized in that it presents one or more absorbent pads in contact with the other end of the chromatographic membrane.

4. Device according to Claim 1, characterized in that it presents a control band set ahead of the said other end of the chromatographic membrane provided with support.

5. Device according to Claim 1, characterized in that the said chromatographic membrane is made of cellulose acetate, nitro-cellulose or nylon.

6. Device according to Claim 1, characterized in that the said marked agent is an immunological component conjugated with coloured substances, and in that the said absorbent pad is made of absorbent paper or glass fabric.

7. Device according to Claim 6, characterized in that the said immunological component is chosen from the group consisting of haptens, antigens and antibodies, and in that the said coloured substances are colloidal particles of gold, silver, platinum, derivatives of metals, sulphur, selenium or tellurium, or else are carbon or coloured latexes.

8. Device according to Claim 1, characterized in that it is in the form of a card or a dipstick.

9. Device according to Claim 1, characterized in that on the chromatographic membrane provided with support it presents an applier for the biological sample to be analyzed, and possibly a filter.

10. Immunochromatographic method for the determination of one or more analytes, comprising:
i) application of a biological sample possibly containing the said analyte or analytes on a device as described in any one of the claims from 1 to 9, in an intermediate area of the chromatographic membrane provided with support;
ii) elution of the marked agent, which is applied to one end of the chromatographic membrane provided with support, using a suitable eluent, which is able to cause the said marked agent to migrate up to the intermediate area in which the analyte has been applied;
iii) detection of the complex resulting from the interaction between the said analyte and the said marked agent, in the intermediate area in which the analyte has been applied.

11. Method according to Claim 10, characterized in that the analyte is chosen from among the group consisting of lipopolysaccharides, protein substances, proteids, drugs, pesticides, polluting substances, and their metabolites.

12. Method according to Claim 11, characterized in that the said protein substances are chosen from among the group consisting of hormones, enzymes, immuno-globulins, allergens, viruses, viral subunits, bacterial extracts, toxins, extracts of mitochondria, and extracts of cell nuclei and cellular membranes, and in that the said proteids are chosen from the group consisting of DNA, RNA, chromosomes and genes.

13. Method according to Claim 12, characterized in that the said hormones are chosen from among the group consisting of HCG, FSH, LH, TSH, T3, T4, and HPRL.

14. Method according to Claim 12, characterized in that the said drugs are chosen from among the group consisting of amphetamines, methamphetamine, barbiturates, benzodiazepines, cannabis-type drugs, marijuana (THC), cocaine, methadone, morphine, heroin, opiates, and phencyclidine.

15. Method according to Claim 10, characterized in that the said analyte consists of the Bence Jones protein, the kappa and lambda chains, or mixtures of these.

16. Method according to Claim 10, characterized in that the said analytes are antibodies specific for HIV and HIV2, HCV, HBV, tuberculosis, mononucleosis, Salmonella, Escherichia coli, H. Pylori, Rubella, Chlamydia, and Streptococcus.

17. Method according to Claim 10, characterized in that the said biological sample is a body fluid chosen from among urine, CSF, blood, plasma and serum, or else consists of mucus or faeces.

18. Method according to Claim 10, characterized in that, after stage (i), the biological sample applied on the device is suitably dried.

19. Method according to Claim 10, characterized in that, in stage (ii), the said eluent is an organic or inorganic buffer containing one or more surfactants.

20. Method according to Claim 19, characterized in that the said sample consists of PBS with physiological pH containing Tween 20 at 0.5-5 wt%, Triton X at 0-5 wt% and PVP at 0-5 wt%.

21. Method according to Claim 10, for the diagnosis of Bence Jones proteinuria.

22. Method according to any one of the claims from 10 to 21, characterized in that, in stage (i), the said biological sample is applied on a number of intermediate bands of the chromatographic membrane provided with support, at progressively increasing dilutions, for semi-quantitative determination of the analyte.

23. Immunochromatographic kit comprising a device as described in any one of the claims from 1 to 9, an appropriate eluent, and, possibly, a positive-control solution and a depositor for application of the sample.
